(19) Europäisches Patentamt
European Patent Office
Office européen des brevets

(11) Veröffentlichungsnummer : **0 345 637 B1**

(12) **EUROPÄISCHE PATENTSCHRIFT**

(45) Veröffentlichungstag der Patentschrift : **17.03.93 Patentblatt 93/11**

(51) Int. Cl.$^5$ : **C07D 405/10,** C07D 409/10, A01N 43/36

(21) Anmeldenummer : **89109913.7**

(22) Anmeldetag : **01.06.89**

# (54) N-substituierte 3,4,5,6-Tetrahydrophthalimid-Derivate.

Die Akte enthält technische Angaben, die nach dem Eingang der Anmeldung eingereicht wurden und die nicht in dieser Patentschrift enthalten sind.

(30) Priorität : **08.06.88 DE 3819464**

(43) Veröffentlichungstag der Anmeldung : **13.12.89 Patentblatt 89/50**

(45) Bekanntmachung des Hinweises auf die Patenterteilung : **17.03.93 Patentblatt 93/11**

(84) Benannte Vertragsstaaten : **CH DE FR GB IT LI**

(56) Entgegenhaltungen :
**EP-A- 0 240 659**
**WO-A-87/07602**
**CHEMICAL ABSTRACTS, Band 109, Nr. 25, 19. Dezember 1988, Columbus, Ohio, US; WHEELER T.N. et al.: "Novel N-[4-chloro-2-fluoro-5-(substituted methyl)phenyl]imides", Seite 830, Spalte 1, Zusammenfassung-Nr. 230 701c**

(56) Entgegenhaltungen :
**CHEMICAL ABSTRACTS, Band 102, Nr. 3, 21. Januar 1985, Columbus, Ohio, US; SUMITOMO CHEMICAL CO., LTD. "4,5,6,7-Tetrahydro-2H-isoindole-1,3-diones", Seite 680, Spalte 1, Zusammenfassung-Nr. 24 478n**
**CHEMICAL ABSTRACTS, Band 105, Nr. 7, 18. August 1986, Columbus, Ohio, US; MATSUMOTO S. et al. "Herbicidal N-substituted dicarboxyimides", Seite 606, Spalte 2, Zusammenfassung-Nr. 60 524v**

(73) Patentinhaber : **BASF Aktiengesellschaft**
**Carl-Bosch-Strasse 38**
**W-6700 Ludwigshafen (DE)**

(72) Erfinder : **Schwalge, Barbara, Dr.**
**Adolf-Diesterweg-Strasse 77**
**W-6700 Ludwigshafen (DE)**
Erfinder : **Plath, Peter, Dr.**
**Hans-Balcke-Strasse 13**
**W-6710 Frankenthal (DE)**
Erfinder : **Eicken, Karl, Dr.**
**Am Hüttenwingert 12**
**W-6706 Wachenheim (DE)**
Erfinder : **Rüb, Lothar, Dr.**
**Zum Weidentor 4**
**W-6720 Speyer (DE)**
Erfinder : **Würzer, Bruno, Dr.**
**Rüdigerstrasse 13**
**W-6701 Otterstadt (DE)**
Erfinder : **Westphalen, Karl-Otto, Dr.**
**Mausbergweg 58**
**W-6720 Speyer (DE)**
Erfinder : **Meyer, Norbert, Dr.**
**Dossenheimer Weg 22**
**W-6802 Ladenburg (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft N-Aryltetrahydrophthalimidverbindungen der allgemeinen Formel I

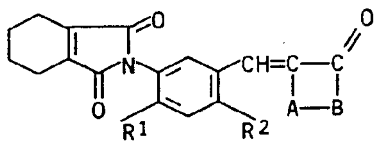

(I),

in der die Substituenten folgende Bedeutung haben:

$R^1$ Wasserstoff oder ein Halogenatom,

$R^2$ ein Halogenatom,

A eine $C_2$-$C_4$-Alkylen- oder $C_2$-$C_4$-Alkenylenbrücke, die ein bis drei $C_1$-$C_3$-Alkylgruppen tragen kann,

B ein Sauerstoff- oder Schwefelatom.

Weiterhin betrifft die Erfindung herbizide Mittel, welche diese Verbindungen enthalten.

Aus der EP-A 0 240 659 sind Verbindungen mit ähnlicher Struktur wie der von I bekannt, die jedoch anstelle des Restes

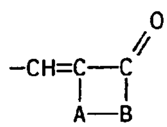

einen offenkettigen Rest tragen.

Besonders für die Anwendung neben Kulturpflanzen, z.B. im Nachauflaufverfahren sind jedoch Verbindungen wünschenswert, die bei geringerer Aufwandmenge höhere Selektivität aufweisen.

Der Erfindung lag daher die Aufgabe zugrunde, geeignete Wirkstoffe zu finden und zu synthetisieren.

Entsprechend dieser Aufgabe wurden die eingangs definierten N-Aryltetrahydrophthalimidverbindungen I gefunden.

Desweiteren wurden Verfahren zur Herstellung der erfindungsgemäßen Verbindungen I sowie ihre Verwendung in herbiziden Mitteln gefunden.

Die substituierten N-(Heterocyclylidenmethyl)-phenyl-3,4,5,6-tetrahydrophthalimid-Derivate der Formel I können aus Tetrahydrophthalsäureanhydrid und einem entsprechenden Anilin-Derivat, z.B. in einem Lösungsmittel bei einer Temperatur von 20 bis 200°C, vorzugsweise 40 bis 150°C, erhalten werden. Als Lösungsmittel eignen sich z.B. niedere Fettsäuren wie Eisessig oder Propionsäure oder aprotische Lösungsmittel. Beim Arbeiten in einem aprotischen Lösungsmittel verwendet man zweckmäßigerweise einen Wasserabscheider.

Die Anilin-Derivate der Formel V können aus den entsprechenden Nitrophenyl-Derivaten IV entweder durch Reduktion mit z.B. Zinn-II-ionen oder Eisen oder durch katalytische Hydrierung an Metallkontakten wie z.B. Raney-Nickel, Palladium oder Platin erhalten werden:

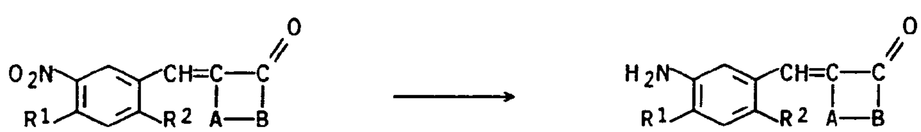

IV V

Die benötigten Nitrophenyl-Derivate IV sind auf verschiedene Weise herstellbar, z.B.:

a) indem man einen entsprechend substituierten Nitrobenzaldehyd der Formel II, in an sich bekannter Weise, z.B. nach den in Synthesis (10), 862 (1984) beschriebenen Bedingungen, in einem Lösungsmittel bei Temperaturen zwischen Raumtemperatur und dem Siedepunkt des Lösungsmittels mit einem Triarylphosphoran der Formel IIIa umsetzt.

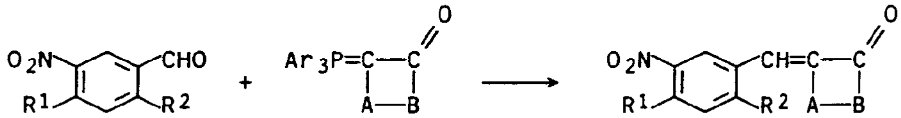

II IIIa IV

Ar in Formel IIIa bedeutet einen aromatischen Rest wie vorzugsweise Phenyl. Als Lösungsmittel eignen sich z.B. Toluol, Tetrahydrofuran, Dimethylformamid, Dimethylsulfoxid und Methanol.

Die benötigten Triarylphosphorane IIIa sind analog zu in der Literatur beschriebenen Verfahren zugänglich (B=O in Helv. Chim. Acta 46, 1580 (1963); B=S in THL 52, 5435 (1968) ).

b) indem man einen entsprechend substituierten Nitrobenzaldehyd II mit einem Phosphonat der Formel IIIb kondensiert.

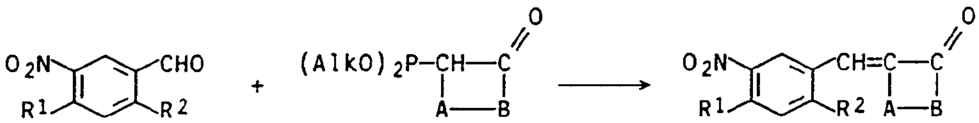

II IIIb IV

Für diese Umsetzung geeignete Bedingungen sind z.B. in Organic Reactions 25, 73 (1977) beschrieben. Man setzt entweder in Gegenwart starker Basen wie z.B. Alkoholat, Metallhydrid oder Metallalkyl in einem aprotischen Lösungsmittel wie Toluol, Tetrahydrofuran, Ether oder Dimethylsulfoxid um oder kondensiert in Zweiphasensystemen mit Zusatz von Phasentransferkatalysator z.B. nach den in Synthesis 1986, 926 beschriebenen Bedingungen. Als Basen eignen sich hier z.B. Carbonate des Natriums oder Kaliums sowie Hydroxide des Natriums, Kaliums, Bariums oder Calciums.

Als Phasentransferkatalysatoren verwendet man hierbei vorzugsweise Kronenether wie 15-Krone-5 oder 18-Krone-6 bzw. entsprechende benzokondensierte Derivate, z.B. Dibenzo-18-Krone-6.

In manchen Fällen können auch Polyethylenglycoldialkylether des Typs

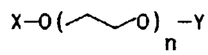

(n=5-7 und X und Y unabhängig voneinander $C_1$-$C_4$-Alkyl) oder quartäre Ammoniumsalze eingesetzt werden.

Man kann aber auch direkt zu Derivaten der Formel I gelangen, wenn man Triarylphosphorane der Formel IIIa mit einem geeignet substituierten Aldehyd VIII unter den bei a) geschilderten Bedingungen kondensiert.

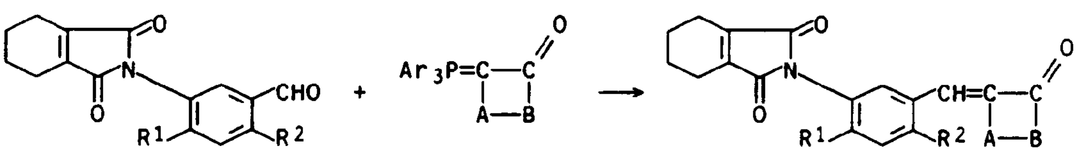

VIII IIIa I

Den hierfür benötigten Aldehyd VIII erhält man, indem man das entsprechende Nitrobenzaldehydacetal [erhältlich gemäß Aust. J. Chem. 23 (10), 2039 (1970)] bei relativ milden Bedingungen an Edelmetallkatalysatoren oder Katalysatoren wie Raney-Nickel hydriert und das so erhaltene Anilin-Derivat VII mit Tetrahydrophthalsäureanhydrid kondensiert.

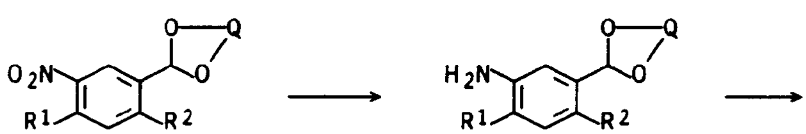

VIII

VI VII

Q in Formel VI bedeutet eine Ethylen- oder Propylenbrücke, welche ein bis drei Alkylgruppen wie vorzugsweise Methyl und Ethyl tragen kann.

Sofern man für die Kondensation als Lösungsmittel niedere Fettsäuren wie z.B. Eisessig oder Propionsäure verwendet, spaltet sich gleichzeitig die Acetalgruppe zum Aldehyd.

Im Hinblick auf die biologische Aktivität sind solche Verbindungen I bevorzugt, in denen die Substituenten folgende Bedeutung haben:

$R^1$ Wasserstoff oder ein Halogenatom wie Fluor, Chlor und Brom, vorzugsweise Fluor,

$R^2$ ein Halogenatom wie bei $R^1$ genannt, vorzugsweise Chlor und Brom;

A Alkylen oder Alkenylen wie Ethylen, Propylen, Butylen, Ethenylen, Propenylen und Butenylen, vorzugsweise Ethylen, Propylen, Ethenylen und Propenylen, wobei diese Reste ein- bis dreifach durch Alkyl wie Methyl, Ethyl, Propyl und 1-Methylethyl, insbesondere Methyl substituiert sein können,

B ein Sauerstoff- oder ein Schwefelatom.

Beispiele für sehr aktive Verbindungen I sind in den nachstehenden Tabellen A und B aufgeführt.

Tabelle A

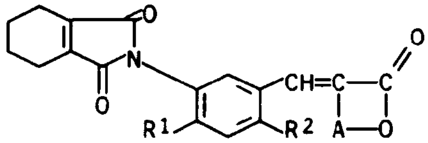

| $R^1$ | $R^2$ | A |
|---|---|---|
| H | Cl | $CH_2CH_2$ |
| F | Cl | $CH_2CH_2$ |
| H | Cl | $CH_2CH(CH_3)$ |
| F | Cl | $CH_2CH(CH_3)$ |
| H | Cl | $CH_2CH(CH_2CH_3)$ |
| F | Cl | $CH_2CH(CH_2CH_3)$ |
| H | Cl | $CH_2CH(CH_2CH_2CH_3)$ |
| F | Cl | $CH_2CH(CH_2CH_2CH_3)$ |
| H | Cl | $CH_2C(CH_3)_2$ |
| F | Cl | $CH_2C(CH_3)_2$ |
| H | Cl | $CH(CH_3)CH_2$ |
| F | Cl | $CH(CH_3)CH_2$ |
| H | Cl | $CH(CH_2CH_3)CH_2$ |
| F | Cl | $CH(CH_2CH_3)CH_2$ |
| H | Cl | $CH(CH_3)CH(CH_3)$ |
| F | Cl | $CH(CH_3)CH(CH_3)$ |
| H | Cl | $C(CH_3)_2CH_2$ |
| F | Cl | $C(CH_3)_2CH_2$ |
| H | Cl | $CH_2C(CH_2CH_3)_2$ |
| F | Cl | $CH_2C(CH_2CH_3)_2$ |
| H | Cl | $CH_2C(CH_3)(C_2H_5)$ |
| F | Cl | $CH_2C(CH_3)(C_2H_5)$ |
| H | Cl | $CH_2CH_2CH_2$ |
| F | Cl | $CH_2CH_2CH_2$ |
| H | Cl | $CH_2CH_2CH(CH_3)$ |
| F | Cl | $CH_2CH_2CH(CH_3)$ |
| H | Cl | $CH_2CH_2C(CH_3)_2$ |
| F | Cl | $CH_2CH_2C(CH_3)_2$ |
| H | Cl | $CH_2CH(CH_3)CH_2$ |
| F | Cl | $CH_2CH(CH_3)CH_2$ |
| H | Cl | $CH_2C(CH_3)_2CH_2$ |
| F | Cl | $CH_2C(CH_3)_2CH_2$ |
| H | Cl | $CH(CH_3)CH_2CH_2$ |
| F | Cl | $CH(CH_3)CH_2CH_2$ |

Tabelle A (Forts.)

| $R^1$ | $R^2$ | A |
|---|---|---|
| H | Cl | $C(CH_3)_2CH_2CH_2$ |
| F | Cl | $C(CH_3)_2CH_2CH_2$ |
| H | Cl | $CH(CH_3)CH(CH_3)CH_2$ |
| F | Cl | $CH(CH_3)CH(CH_3)CH_2$ |
| H | Cl | $CH_2CH(CH_3)CH(CH_3)$ |
| F | Cl | $CH_2CH(CH_3)CH(CH_3)$ |
| H | Cl | $CH(CH_3)CH(CH_3)CH(C_2H_5)$ |
| F | Cl | $CH(CH_3)CH(CH_3)CH(C_2H_5)$ |
| H | Cl | $CH_2CH_2CH(C_3H_7)$ |
| F | Cl | $CH_2CH_2CH(C_3H_7)$ |
| H | Cl | $CH_2CH_2CH_2CH_2$ |
| F | Cl | $CH_2CH_2CH_2CH_2$ |
| H | Cl | $CH=CH$ |
| F | Cl | $CH=CH$ |
| H | Cl | $CH=C(CH_3)$ |
| F | Cl | $CH=C(CH_3)$ |
| H | Cl | $CH=C(C_2H_5)$ |
| F | Cl | $CH=C(C_2H_5)$ |
| H | Cl | $CH=CHCH_2$ |
| F | Cl | $CH=CHCH_2$ |
| H | Cl | $CH=C(CH_3)CH_2$ |
| F | Cl | $CH=C(CH_3)CH_2$ |
| H | Cl | $CH_2C(CH_3)=CH$ |
| F | Cl | $CH_2C(CH_3)=CH$ |
| H | Cl | $CH_2CH=CH$ |
| F | Cl | $CH_2CH=CH$ |
| H | Cl | $CH(CH_3)CH=CH$ |
| F | Cl | $CH(CH_3)CH=CH$ |
| H | Cl | $C(CH_3)_2CH=CH$ |
| F | Cl | $C(CH_3)_2CH=CH$ |
| H | Cl | $CH=CHCH_2CH_2$ |
| F | Cl | $CH=CHCH_2CH_2$ |

Tabelle B

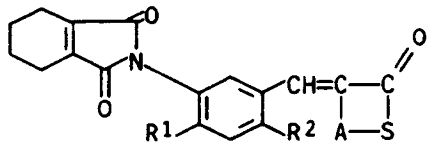

| $R^1$ | $R^2$ | A |
|---|---|---|
| H | Cl | $CH_2CH_2$ |
| F | Cl | $CH_2CH_2$ |
| H | Cl | $CH_2CH(CH_3)$ |
| F | Cl | $CH_2CH(CH_3)$ |
| H | Cl | $CH(CH_3)CH_2$ |
| F | Cl | $CH(CH_3)CH_2$ |
| H | Cl | $CH(CH_3)CH(CH_3)$ |
| F | Cl | $CH(CH_3)CH(CH_3)$ |
| H | Cl | $CH_2CH_2CH_2$ |
| F | Cl | $CH_2CH_2CH_2$ |
| H | Cl | $CH(CH_3)CH_2CH_2$ |
| F | Cl | $CH(CH_3)CH_2CH_2$ |
| H | Cl | $CH_2CH_2CH_2CH_2$ |
| F | Cl | $CH_2CH_2CH_2CH_2$ |
| H | Cl | $CH=CH$ |
| F | Cl | $CH=CH$ |
| H | Cl | $CH=C(CH_3)$ |
| F | Cl | $CH=C(CH_3)$ |
| H | Cl | $CH=C(C_2H_5)$ |
| F | Cl | $CH=C(C_2H_5)$ |
| H | Cl | $CH_2CH=CH$ |
| F | Cl | $CH_2CH=CH$ |
| H | Cl | $CH_2C(CH_3)=CH$ |
| F | Cl | $CH_2C(CH_3)=CH$ |
| H | Cl | $CH_2CH_2CH_2CH_2$ |
| F | Cl | $CH_2CH_2CH_2CH_2$ |

Die N-Aryltetrahydrophthalimidverbindungen I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 1.001 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 1.003 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 2.001 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.002 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 2.001 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.001 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.002 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 2.001 werden mit 2 Gewichtsteilen Calciumsalz der

Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lag-by).

Die Aufwandmengen an Wirkstoff betragen je nach Bekämpfungsziel, Jahreszeit, Zielpflanzen und Wachstumsstadium 0,001 bis 5,0, vorzugsweise 0,01 bis 1,0 kg/ha aktive Substanz (a.S.).

Die N-Aryltetrahydrophthalimide der allgemeinen Formel I können daneben praktisch alle Entwicklungsstadien einer Pflanze verschiedenartig beeinflussen und werden deshalb auch als Wachstumsregulatoren eingesetzt. Die Wirkungsvielfalt der Verbindungen hängt von verschiedenen Faktoren ab. Maßgeblich sind dabei vor allem

a) Pflanzenart und -sorte,

b) Zeitpunkt der Applikation, bezüglich Entwicklungsstadium der Pflanze und Jahreszeit,

c) Applikationsort und -verfahren (Samenbeize, Bodenbehandlung oder Blattapplikation)

d) klimatische Faktoren, z.B. Temperatur, Niederschlagsmenge, außerdem auch Tageslänge und Lichtintensität

e) Bodenbeschaffenheit (einschließlich Düngung),

f) Formulierung bzw. Anwendungsform des Wirkstoffs und

g) Aufwandmenge und Wirkstoffgehalt der Mittel.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, sind nachstehend einige Beispiele aufgeführt.

A. Mit den wachstumsregulierenden N-Aryltetrahydrophthalimiden I läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Bei Obstgehölzen und anderen Bäumen oder Sträuchern können dadurch kostenintensive Schnittmaßnahmen reduziert werden.

Von wirtschaftlichem Interesse ist auch die Erhöhung der Standfestigkeit von lageranfälligen Kulturen wie Getreide, Reis, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung zur Hemmung des Längenwachstums und zur zeitlichen Veränderung des Reifeverlaufs bei Baumwolle. Damit wird ein vollständig mechanisiertes Beernten dieser wichtigen Kulturpflanzen ermöglicht.

Durch Anwendung der Verbindungen I kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit den Verbindungen I läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag, bezogen auf die Bodenfläche, erzielt werden kann.

B. Mit den wachstumsregulierenden Mitteln auf der Basis von N-Aryltetrahydrophthalimiden I lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Die N-Aryltetrahydrophthalimide der Formel I können Ertragssteigerungen durch Eingriffe in den pflanzlichen Stoffwechsel bzw. durch Förderung oder Hemmung des vegetativen und/oder des generativen Wachstums verursachen.

C. Mit den N-Aryltetrahydrophthalimiden I lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Die Förderung der Ausbildung eines Trenngewebes zwischen der Blatt- und Sproßachse ist auch für ein gut kontrollierbares Entblättern von Nutzpflanzen z.B. Baumwolle wesentlich.

D. Mit den N-Aryltetrahydrophthalimiden I kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird
- das Mikroklima im Pflanzenbestand durch einen kompakteren Wuchs günstig beeinflußt wird.

Die erfindungsgemäß zu verwendenden Wirkstoffe können den Kulturpflanzen sowohl vom Samen her (als Saatgutbeizmittel) als auch über den Boden, d.h. durch die Wurzel sowie durch Spritzung über das Blatt zugeführt werden.

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |

| Botanischer Name | Deutscher Name |
|---|---|
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |

| Botanischer Name | Deutscher Name |
|---|---|
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die N-Aryltetrahydrophthalimide I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt.

Beispiel 1

N-[4-Chlor-3-(4′-methyl-2′-oxo-3′-oxa-cyclopentylidenmethyl]-phenyl-3.4.5.6-tetrahydrophthalimid

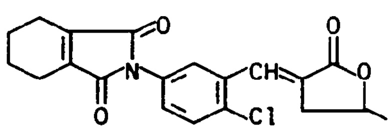

a) Zu einer Mischung aus 7.4 g (0,054 mol) Kaliumcarbonat und 3,5 g (0,0135 mol) 18-Krone-6 in 10 ml abs. Tetrahydrofuran gibt man bei 0-5°C unter Rühren 7,1 g (0,03 mol) 4-Methyl-2-oxo-3-oxa-cyclopentyl-diethylphosphonat [Z. Naturforsch. B. 38B (4), 493 (1983)] in 8 ml abs. Tetrahydrofuran. Nach ca. 0,5 h versetzt man mit 5,0 g (0,027 mol) 2-Chlor-5-nitrobenzaldehyd in 6 ml abs. Tetrahydrofuran und läßt 15 h bei RT nachrühren. Zur Aufarbeitung gießt man auf 40 ml Eiswasser und extrahiert mehrfach mit $Et_2O$. Nach Waschen der org. Phase mit 10 %iger HCl und $H_2O$, Trocknen und Eindampfen des Lösungsmittels wird das Produkt an Kieselgel mit Toluol/Aceton (9:1) als Eluens getrennt. Man erhält so 1,7 g 2-(4′-Methyl-2′-oxo-3′-oxa-cyclopentylidenmethyl)-4-nitro-chlorbenzol. (Fp. 87-103°C, Isomerengemisch).

b) Zu einer Mischung aus 3,0 g (0,054 mol) Eisenpulver, 7,5 ml Eisessig und 15 ml Methanol gibt man unter Rühren bei 60°C innerhalb 15 min 2,4 g (0,009 mol) 2-(4′-Methyl-2′-oxo-3′-oxa-cyclopentylidenmethyl)-4-nitro-chlorbenzol in 10 ml Eisessig und 10 ml Methanol. Nach beendeter Zugabe wird 30 min unter Reflux nachgerührt, auf RT abgekühlt, abfiltriert und das Lösungsmittel im Vakuum abgezogen. Der Rückstand wird mit Essigester aufgenommen, zweimal mit $NaHCO_3$-Lösung und zweimal mit $H_2O$ gewaschen, über $MgSO_4$ getrocknet und einrotiert. Man erhält so 2,3 g rohes 4-Amino-2-(4′-methyl-2′-oxo-3′-oxa-cyclopentylidenmethyl)-chlorbenzol welches ohne weitere Aufarbeitung umgesetzt wird.

c) 2,2 g (0,009 mol) 4-Amino-2-(4′-methyl-2′-oxo-3′-oxa-cyclopentylidenmethyl)-chlorbenzol, 1,4 g (0,009

mol) 3.4.5.6-Tetrahydrophthalsäureanhydrid und 25 ml Eisessig werden gemeinsam 2 h unter Rückfluß gerührt. Nach dem Abkühlen auf RT wird das Lösungsmittel abgezogen, in 100 ml Essigester aufgenommen, je zweimal mit $NaHCO_3$-Lösung und $H_20$ gewaschen, über $MgSO_4$ getrocknet und einrotiert. Man erhält so 3,5 g Rohprodukt, welches nach Chromatographie an Kieselgel mit Toluol:Aceton (98:2) 2,5 g N-[4-Chlor-3-(4′-methyl-2′-oxo-3′-oxa-cyclopentylidenmethyl]-phenyl]-3.4.5.6-tetrahydrophthalimid liefert. (Fp. 98-113°C; Wirkstoffbeispiel 1.003)

Beispiel 2

N-[4-Chlor-3-(2′-oxo-3′-oxa-cyclopentylidenmethyl)]-phenyl-3.4.5.6-tetrahydrophthalimid

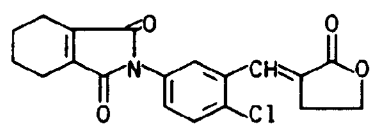

a) Man erhitzt ein Mischung aus 3,4 g (0,01 mol) 2-Oxo-3-oxa-cyclopentyliden-triphenylphosphoran [Helv. Chim. [Acta. 46, 1580 (1963)] und 1,6 g (0,01 mol) 2-Chlor-5-nitrobenzaldehyd in 150 ml abs. Dimethylformamid unter Rühren 18 h auf 110°C. Nach dem Abkühlen gibt man auf 0,5 l Eiswasser und filtriert den gebildeten Niederschlag ab. Nach dem Trocknen erhält man 1,1 g 4-Nitro-2-(2′-oxo-3′-oxa-cyclopentylidenmethyl)-chlorbenzol. (Fp.: 157-161°C)

b) 3,1 g (0,012 mol) 4-Nitro-2-(2′-oxo-3′-oxa-cyclopentylidenmethyl)-chlorbenzol werden mit 2,3 g Eisenpulver in insgesamt 14 ml Eisessig und 45 ml Methanol analog Beispiel 1b) reduziert. Nach der Aufarbeitung erhält man 2,1 g 4-Amino-2-(2′-oxo-3′-oxa-cyclopentylidenmethyl)-chlorbenzol als Öl, welches ohne weitere Reinigung umgesetzt wird.

c) 2,4 g (0,011 mol) 4-Amino-2-(2′-oxo-3′-oxa-cyclopentylidenmethyl)-chlorbenzol und 1,6 g (0,011 mol) 3.4.5.6-Tetrahydrophthalsäureanhydrid werden in 25 ml Essigsäure (konz.) gemeinsam 5 h unter Rühren refluxiert. Nach dem Abkühlen filtriert man das Produkt ab, wäscht mit wenig $H_2O$ und $NaHCO_3$-Lösung und trocknet. Man erhält so 2,3 g N-[4-Chlor-3-(2′-oxo-3′-oxa-cyclopentylidenmethyl)]-phenyl-3.4.5.6-tetrahydrophthalimid. (Fp.: 176-180°C; Wirkstoffbeispiel 1.001)

Beispiel 3

N-[4-Chlor-3-(2′-oxo-3′-thia-cyclopentylidenmethyl)]-phenyl-3.4.5.6-tetrahydrophthalimid

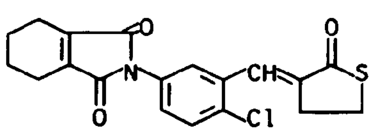

a) 371 g (2,00 mol) 2-Chlor-5-nitro-benzaldehyd, 137 g (2,20 mol) Ethylenglykol und 1,00 g p-Toluolsulfonsäure werden in 1 500 ml Toluol 5 Stunden am Wasserauskreiser zum Rückfluß erhitzt. Nach Abkühlung auf Raumtemperatur und Einengen im Vakuum verbleiben 459 g (100 %) 2-(2-Chlor-5-nitro)-phenyl-1.3-dioxolan vom Schmp. 88-90°C.

b) 115 g (0,50 mol) der in Beispiel 3a) dargestellten Verbindung werden in 1 000 ml THF nach Zugabe von 20 g Raney-Nickel bei einem Überdruck von 0,05 bar und einer Temperatur von 50 °C hydriert. Ausbeute: 99,0 g (99 %) 2-(5-Amino-2-chlor)-phenyl-1,3-dioxolan (Öl).

c) 99,8 g (0,50 mol) Produkt aus Beispiel 3b) und 76,1 g (0,50 mol) 3.4.5.6-Tetrahydrophthalsäureanhydrid werden in 500 ml Eisessig 5 Stunden zum Rückfluß erhitzt. Nach Abkühlung auf Raumtemperatur wird mit 500 ml Wasser versetzt, der gebildete Niederschlag abgesaugt, mit $H_2O$ gewaschen und getrocknet. Ausbeute: 120 g (83 %) N-(4-Chlor-3-formyl)-phenyl-3.4.5.6-tetrahydrophthalimid vom Schmp. 140-141°C.

d) Man erhitzt eine Mischung aus 3,6 g (0,01 mol) 2-Oxo-3-thia-cyclopentyliden-triphenylphosphoran [THL 52, 5435 (1968)] und 2,9 g (0,01 mol) N-(4-Chlor-3-formyl)-phenyl-3.4.5.6-tetrahydrophthalimid in 100 ml abs. Methanol 8 h unter Rühren auf Reflux. Nach dem Abkühlen wird das Lösungsmittel abgezogen und der Rückstand an Kieselgel mit Toluol/Cyclohexan (9:1) als Eluent getrennt. Man erhält so 1,3 g N-[4-Chlor-3-(2′-oxo-3′-thia-cyclopentylidenmethyl)]-phenyl-3.4.5.6-tetrahydrophthalimid (gelbes Öl; Wirkstoffbeispiel 2.001).

Beispiel 4

N-[4-Chlor-2-fluor-5-(2′-oxo-3′-oxa-cyclopentylidenmethyl)]-phenyl-3.4.5.6-tetrahydrophthalimid

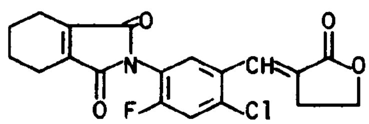

a) Analog den Beispielen 3a) - c) kann 2-Chlor-4-fluor-5-nitro-benzaldehyd durch säurekatalysierte Umsetzung mit Propan-1.3-diol in 2-(2-Chlor-4-fluor-5-nitro)-phenyl-1.3-dioxan überführt werden (Schmp. 104-105°C), das sich in Gegenwart von Raney-Nickel zum 2-(5-Amino-2-chlor-4-fluor)-phenyl-1.3-dioxan (Schmp. 80-82°C) reduzieren läßt. Die anschließende Kondensation mit 3.4.5.6-Tetrahydrophthalsäureanhydrid in Eisessig ergibt das gewünschte N-(4-Chlor-2-fluor-5-formyl)-phenyl-3.4.5.6-tetrahydrophthalimid (Schmp.: 131-132°C).

b) Analog Beispiel 3d) erhält man aus 3,5 g (0,01 mol) 2-Oxo-3-oxa-cyclopentyliden-triphenylphosphoran und 3,1 g (0,01 mol) N-(4-Chlor-2-fluor-5-formyl)-phenyl-3.4.5.6-tetrahydrophthalimid nach 5 h bei Reflux rühren in 100 ml Methanol (abs) und Chromatographie an Kieselgel mit Toluol/Cyclohexan (9:1) 1,6 g N-[4-Chlor-2-fluor-5-(2′-oxo-3′-oxa-cyclopentylidenmethyl)]-phenyl-3.4.5.6-tetrahydrophthalimid (Fp.: 89-118°C; Wirkstoffbeispiel 1.002).

Tabelle 1

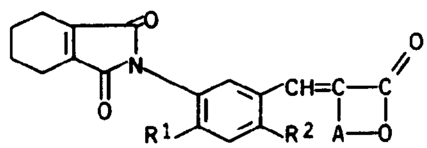

| Nr. | $R^1$ | $R^2$ | A | Fp. |
|---|---|---|---|---|
| 1.001 | H | Cl | $CH_2CH_2$ | 176-180°C |
| 1.002 | F | Cl | $CH_2CH_2$ | 89-118°C |
| 1.003 | H | Cl | $CH_2CH(CH_3)$ | 98-113°C |

Tabelle 2

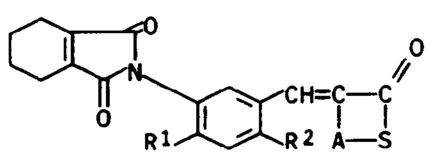

| Nr. | $R^1$ | $R^2$ | A | Fp. |
|---|---|---|---|---|
| 2.001 | H | Cl | $CH_2CH_2$ | öl |

Anwendungsbeispiele

Die herbizide Wirkung der N-Aryltetrahydrophthalimide der Formel I ließ sich durch Gewächshausversuche zeigen:

Zur Aufzucht der Testpflanzen dienten Plastikblumentöpfe mit 300 $cm^3$ Inhalt und lehmigem Sand mit etwa 3 % Humus als Substrat. Die Samen der Testpflanzen wurden nach Arten getrennt flach eingesät.

Bei Vorauflaufbehandlung wurden die aufbereiteten Wirkstoffe unmittelbar danach auf die Erdoberfläche aufgebracht. Sie wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen. Danach wurden die Gefäße mit durchsichtigen Plastikhauben abgedeckt, bis die Pflanzen angewaschsen waren. Diese Abdeckung fördert ein gleichmäßiges Keimen der Testpflanzen, sofern dies nicht durch die Wirkstoffe beeinträchtigt wird.

Zum Zwecke der Nachauflaufbehandlung wurden die Testpflanzen je nach Wuchsform erst bis zu einer Wuchshöhe von 3 bis 15 cm angezüchtet und erst dann mit den in Wasser suspendierten oder emulgierten Wirkstoffen behandelt. Die Testpflanzen wurden dafür entweder direkt gesät und in den gleichen Gefäßen aufgezogen oder sie wurden als Keimpflanzen getrennt gezüchtet und einige Tage vor der Behandlung in die Versuchsgefäße verpflanzt. Die Aufwandmengen für die Nachauflaufbehandlung betrugen 0,125 und 0,06 kg Wirkstoff/ha. Eine Abdeckung unterblieb bei der Nachauflaufbehandlung.

Die Versuchsgefäße wurden im Gewächshaus aufgestellt, wobei für wärmeliebende Arten wärmere Bereiche (20 bis 35°C) und für solche gemäßigter Klimate 10 bis 25°C bevorzugt wurden. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürzung | Lateinischer Name | Deutscher Name |
|---|---|---|
| ABUTH | Abutilon theophrasti | chinesischer Hanf |
| AMARE | Amaranthus retroflexus | zurückgekrümmter Fuchsschwanz |
| CASTO | Cassia tora | - |
| CHEAL | Chenopodium album | weißer Gänsefuß |
| CHYCO | Chysanthemum corinarium | Kronenwucherblume |
| GALAP | Galium aparine | Klettenlabkraut |
| SOLNI | Solanum nigrum | Schwarzer Nachtschatten |
| STEME | Stellaria media | Vogelsternmiere |
| TRZAS | Triticum aestivum | Sommerweizen |
| TRZAW | Triticum aestivum | Winterweizen |

Mit 0,06 kg/ha s.A. im Nachauflaufverfahren eingesetzt, lassen sich mit Beispiel 1.001 und 1.003 unerwünschte Pflanzen sehr gut bekämpfen, und zwar mit gleichzeitiger Verträglichkeit für Sommerweizen.

Dergleichen hat Verbindung Nr. 1.001 mit 0,125 kg/ha a.S. bei Nachauflaufanwendung herbizide Wirkung gegen Stellaria media und Galium aparine.

## Patentansprüche

1. N-Aryltetrahydrophthalimidverbindungen der allgemeinen Formel I

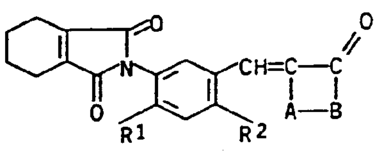

I,

in der die Substituenten folgende Bedeutung haben:

$R^1$ Wasserstoff oder ein Halogenatom,

$R^2$ ein Halogenatom,

A eine $C_2$-$C_4$-Alkylen- oder $C_2$-$C_4$-Alkenylenbrücke, die ein bis drei $C_1$-$C_3$-Alkylgruppen tragen kann,

B ein Sauerstoff- oder Schwefelatom .

2. Verwendung von Verbindungen der Formel I, gemäß Anspruch 1, als Herbizide.

3. Herbizide Mittel, enthaltend eine Verbindung der Formel I gemäß Anspruch 1 und übliche Hilfsstoffe, Streck- und Verdünnungsmittel.

4. Herbizide Mittel gemäß Anspruch 3, enthaltend eine Verbindung der Formel I und weitere wirksame Bestandteile.

5. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge einer N-Aryltetrahydrophthalimidverbindung I gemäß Anspruch 1 behandelt.

6. Verfahren zur Beeinflussung des Pflanzenwachstums, dadurch gekennzeichnet, daß man die Pflanzen, deren Samen oder deren Lebensraum mit einer wachstumsregulativ wirksamen Menge einer N-Aryltetrahydrophthalimidverbindung I gemäß Anspruch 1 behandelt.

## Claims

1. An N-aryltetrahydrophthalimide compound of the general formula I

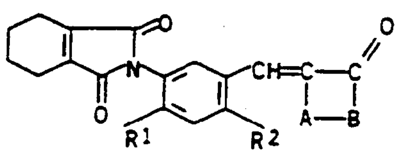

I

where $R^1$ is hydrogen or halogen, $R^2$ is halogen, A is a $C_2$-$C_4$-alkylene or $C_2$-$C_4$-alkenylene bridge which may carry from one to three $C_1$-$C_3$-alkyl groups, and B is oxygen or sulphur.

2. Use of a compound of the formula I as claimed in claim 1 as a herbicidal agent.

3. A herbicidal agent containing a compound of the formula I as claimed in claim 1, and conventional auxiliaries, extenders and diluents.

4. A herbicidal agent as claimed in claim 3, containing a compound of the formula I and other active constituents.

5. A method for controlling the growth of undesirable plants, wherein the undesirable plants and/or their habitat are treated with a herbicidally effective amount of an N-aryltetrahydrophthalimide compound I as claimed in claim 1.

6. A method for influencing plant growth, wherein the plants, their seed or their habitat are treated with an amount effecting growth regulation of an N-aryltetrahydrophthalimide compound I as claimed in claim 1.

## Revendications

1. Dérivés de N-aryltétrahydrophtalimide de la formule générale I

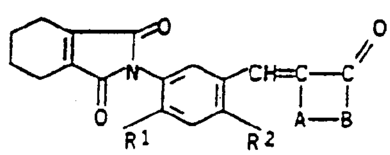

I,

dans laquelle les substituants ont les significations suivantes :

$R^1$, hydrogène ou un atome d'halogène

$R^2$, un atome d'halogène

A, un alkylène en C2-C4 ou un pont alcényle en C2-C4 qui peut porter un à trois groupes alkyle en C1-C3,

B, un atome d'oxygène ou de soufre

**2.** Utilisation de dérivés de formule I selon la revendication 1 comme herbicides.

**3.** Herbicide, contenant un dérivé de formule I selon la revendication 1 et des auxiliaires, agents d'étirage et diluants usuels.

**4.** Herbicide selon la revendication 3, contenant un dérivé de formule I et d'autres constituants actifs.

**5.** Procédé de lutte contre la croissance indésirable de plantes, caractérisé par le fait que l'on traite les plantes indésirables et/ou leur biotope avec une quantité active au point de vue herbicide d'un dérivé de N-aryltétrahydrophtalimide I selon la revendication 1.

**6.** Procédé pour influer sur la croissance des plantes, caractérisé par le fait que l'on traite les plantes, leurs semences ou leur biotope avec une quantité efficace au point de vue régulation de croissance d'un dérivé de N-aryltétrahydrophtalimide I selon la revendication 1.